# EUROPÄISCHE PATENTSCHRIFT

(11) **EP 0 305 758 B1**
(45) Veröffentlichungstag und Bekanntmachung des Hinweises auf die Patenterteilung: **22.12.1993**
(21) Anmeldenummer: 88112632.0
(22) Anmeldetag: 03.08.1988
(51) Int. Cl.: A61L 15/58, A61L 15/44

(54) **Verfahren zur Herstellung eines Pflasters zur gesteuerten Abgabe von Nitroglycerin an die Haut.**
Method for the preparation of a patch for the transdermal sustained release of nitroglycerine
Procédé de préparation d'un pansement pour la libération transdermale retardée de la nitroglycérine

(30) Priorität: 01.09.1987 DE 3729165; 23.12.1987 DE 3743946
(43) Veröffentlichungstag der Anmeldung: 08.03.1989
(73) Patentinhaber: LTS Lohmann Therapie-Systeme GmbH & Co. KG, 56513 Neuwied (DE)
(72) Erfinder: Jaeger, Halvor, D-7910 Neu-Ulm (DE); Hoffmann, Hans-Rainer, D-5450 Neuwied 22 (DE); Meconi, Reinhold, D-5451 Neuwied 11 (DE); Klein, Robert-Peter, D-5450 Neuwied 11 (DE)
(74) Vertreter: Neidl-Stippler, Cornelia, Dr.

(56) Entgegenhaltungen:
- EP-A- 0 086 468
- EP-A- 0 127 282
- EP-A- 0 144 486
- EP-A- 0 186 019
- WO-A-88/01516
- US-A- 4 515 909

## Beschreibung

Die Erfindung betrifft ein Verfahren zur Herstellung eines Pflasters zur gesteuerten Abgabe von Nitroglycerin an die menschliche oder tierische Haut mit einer für das Nitroglycerin undurchlässigen Rückschicht und einem haftklebenden Nitroglycerin enthaltenden Reservoir.

Nitroglycerinpflaster sind bereits bekannt. Die Herstellung von Nitroglycerinpflastern ist insofern problematisch, als Nitroglycerin explosiv ist und infolgedessen seine Verdampfung möglichst vermieden werden sollte.

Es ist bevorzugt, bei möglichst niedrigen Temperaturen, insbesondere bei Raumtemperatur, zu arbeiten, wobei häufig die nitroglycerinhaltige Haftkleberschicht aus der Lösung hergestellt wurde. Die derart hergestellten bekannten pflasterartigen transdermalen therapeutischen Systeme zur Abgabe von Nitroglycerin erfüllten durchaus die therapeutischen Aufgaben, waren aber in der Herstellung zu aufwendig.

In der DF-OS 32 22 800 (ALZA) ist ein Nitroglycerinpflaster beschrieben, wobei als nitroglycerinhaltige Matrix eine bei Raumtemperatur durch Andickung einer Nitroglycerinlösung mit einem rheologischen Mittel erhältliche nicht klebende viskose Masse eingesetzt wird. Auch im US-A-3,742,951 (CIBA-GEIGY) sowie der DE-PS 33 15 272 und DE-OS 33 15 245 (LOHMANN/SCHWARZ) werden einfach aufgebaute Nitroglycerinpflaster mit bei Raumtemperatur aus der Lösung hergestellten haftklebenden Matrixmaterialien beschrieben. Es ist auch bereits bekannt, so aus der DE-OS 36 42 931 (CIBA-GEIGY) mehrteilige Nitroglycerinreservoirs einzusetzen.

Die Verwendung von Lösemitteln bei der Haftkleberschicht herstellung ist aber aus mehreren Gründen nachteilig. Die Herstellung der Lösungen erfordert mindestens einen weiteren aufwendigen Verfahrensschritt. Sie bedingt einen hohen technischen Aufwand und zusätzliche Kosten für die Handhabung, für medizinische Zwecke müssen hochreine und damit teure Lösemittel für die Auflösung der Kleber bzw. deren Ausgangsmaterialien eingesetzt werden, um eine entsprechende Rückstandsfreiheit im transdermalen System sicherzustellen. Ein weiteres Problem besteht darin, Lösemittelfreiheit im Pflaster zu erreichen, wofür teure Trocknungsstrecken und Absauganlagen erforderlich sind. Zusätzlich treten Kosten durch Wiedergewinnung und Abscheidung der Lösemittel auf, um Umweltbelastung zu vermeiden. Daneben stellt die Brennbarkeit der Lösemittel, insbesondere beim hier vorliegenden explosiven Wirkstoff ein zusätzliches Risiko dar. Die meisten organischen Lösemittel sind ferner schädlich für den menschlichen Organismus, so daß aufwendige Schutzmaßnahmen für die in Betrieb tätigen Personen getroffen werden müssen.

Es ist somit Aufgabe der Erfindung, die oben aufgeführten Nachteile der gattungsgemäßen Verfahren nach dem Stand der Technik zu vermeiden.

Die Aufgabe wird erfindungsgemäß durch ein Verfahren zur Herstellung eines Pflasters zur gesteuerten Abgabe von Nitroglycerin an die menschliche oder tierische Haut mit einer für das Nitroglycerin undurchlässigen Rückschicht und einem haftklebenden Nitroglycerin enthaltenden Reservoir gelöst, das die Herstellung einer Mischung aus geschmolzenem Haftschmelzkleber mit einer Verarbeitungstemperatur von 40 bis 80°C und Nitroglycerin und Aufbringen der Mischung auf eine Unterlage aufweist.

Dabei kann das Nitroglycerin im Haftschmelzkleber an einem Träger, wie Lactose, adsorbiert, oder auch in einem Bestandteil des Haftschmelzklebers gelöst, eingesetzt werden.

Bevorzugt kann die Verarbeitungstemperatur des Haftschmelzklebers zwischen 40 und 60°C und besonders bevorzugt zwischen 40 und 55°C liegen.

Vorteilhafte Weiterbildungen der Erfindung ergeben sich aus den Unteransprüchen.

Dadurch, daß bei der Herstellung des Nitroglycerin-Reservoirs bei niedrigen Temperaturen ohne Lösemittel gearbeitet werden kann, ist eine erhebliche Ersparnis an Materialien, eine schnellere Herstellung ohne zeitaufwendige Trocknungsschritte sowie eine die Umwelt weniger belastende Herstellung möglich, was unter anderem zu einem erheblich kostengünstigeren Produkt bei einem ungefährlicheren Herstellungsverfahren führt.

Unter Haftschmelzkleber wird jeder Haftklebstoff verstanden, der heiß hinreichend flüssig ist, um problemlos bei einer Temperatur oberhalb etwa 40°C aufgetragen zu werden.

Als erfindungsgemäß einsetzbare Haftschmelzkleber können u.a. solche eingesetzt werden, die dem Fachmann geläufig sind und wie sie u.a. aus den DE-A-15 94 268 (SUN OIL CO.), DE-A-24 13 979 (E.I. DU PONT DE NEMOURS), DE-A-24 35 863 (DYNAMIT NOBEL AG); DE-A-28 00 302 (CIBA GEIGY); den EP-A- 104 005 (PERSONAL PRODUCTS CO.), den JP 6104 2583 und JP 61 281 810, der EP-A-131 460 (EXXON) und der EP-A 234 856 (EXXON), der EP-A-185 992 (EASTMAN KODAK) sowie der US-A-36 99 963 und der US-A-4,358,557 (EASTMAN KODAK) herleitbar sind, wobei zur Vermeidung von Wiederholungen auf diesen Stand der Technik ausdrücklich Bezug genommen wird.

Als Grundpolymere können bspw. Polyamide, Polyester, Polycaprolactame, Polycaprolacton, Ethylen-Vinylacetat-Copolymere (EVA), Ethylen-Ethylacrylat-Copolymere (EEA), Polyvinylether, Polyacrylatester, Polyvinylacetale, Polyvinylacetate, Styrol-Butadien-Blockpolymere, Isopren-Blockpolymere, Polyurethane, Ethylcellulose, Celluloseacetat- butyrat, Synthesekautschuke (z.B. Neopren-Kautschuk), Polyisobutylen, Butylgummi, Acrylnitril-Butadien-Mischpolymerisate, Epoxidharze, Melaminharze, Phenol-Formaldehyd-Harze und Resorcin-Formaldehyd-Harze verwendet werden, wobei auch unter anderem die nachfolgend genannten modifizierenden Harze eingesetzt werden können: hydriertes Kolophonium, polymerisiertes Kolophonium, dimerisierte Harzsäuren, disproportioniertes Kolophonium, Methylester von Kolophonium, Glycerinester von hydriertem Kolophonium, Methylester von hydriertem Kolophonium, Pentalester, Triethylenglykolester von hydriertem Kolophonium, Hydro abiethylalkohol und dessen Derivate, Glycerinester, Di-, Triolester und Pentaester von Harzsäuren, Pentalester von polymerisiertem Kolophonium, Pentalester von dimerisiertem Kolophonium, Glycerinester von dimerisiertem Kolophonium, Ester von Maleinsäure- oder Phenol-modifiziertem Kolophonium, aromatische und aliphatische Kohlenwasserstoffharze, hydrierte Harze, Polyterpenharze modifizierte Terpenharze, Wachse, niedermolekulares Polyethylen und Polypropylen, Alkyl-Styrol-Copolymere. Diesen Harzen können ggf Weichmacher, wie z.B. Adipinsäureester, Phosphorsäureester, Phthalsäureester, Polyester, Fettsäureester, Citronensäureester oder Epoxidweichmacher zugesetzt werden. Außerdem können auch Stabilisatoren, wie Tocopherol, substituierte Phenole, Hydrochinone, Brenzkatechine, aromatische Amine, und ggf. auch noch Füllstoffe, wie z. B. Titandioxid, Magnesiumoxid, Zinkoxid und Siliciumdioxid, zugemischt werden.

Die Bildung der Bestandteile des Nitroglycerinpflasters, die Haftschmelzkleber mit einer Verarbeitungstemperatur zwischen 40 und 80°C aufweisen, kann durch Extrusion, Gießen, Walzenauftrag, Rakelauftrag, Aufsprühen oder ein Druckverfahren erfolgen.

Ein Grenzwert für die Verarbeitbdrkeit der Haftschmelzkleder bei vielen dieser Verfahren ist bei einer Viskosität im Bereich von etwa 80 000 Pa gegeben.

Falls die mit dem Kleber zu behandelnde Unterlage - eine Komponente der Vorrichtung - durch die Temperatur des heiß aufgetragenen Klebers beschädigt werden könnte - sei es durch Zersetzung, Reaktion oder partielles Schmelzen, kann eine gekühlte Unterlage eingesetzt werden. Die Kühlung kann durch an sich bekannte Verfahren erfolgen, wie durch Einbringung kalter inerter Gase oder das Kontaktieren mit einer Kühlfläche.

Der Haftschmelzkleber kann bspw. in Schichtform oder in einzelnen Bereichen entsprechend einem vorherbestimmten Muster auf die Schutzschicht oder das Abdeckmaterial aufgetragen werden.

Typische Zusammensetzungen für einzusetzende Haftschmelzklebstoffe sind solche, die aus zwischen 10 und 80 Gew.%, bevorzugt 20 bis 80 Gew.% und besonders bevorzugt 20 bis 50 Gew.% Polymer, zwischen 1 und 80 Gew.% Weichmacher; zwischen 10 und 80 Gew.%, bevorzugt 15 bis 60 Gew.% Tackifier, ggf. 0.1 bis 5 Gew.% Alterungsschutzmittel und ggf. 0 bis 70 Gew.% Füllstoffe hergestellt sind, wobei die Summe der Prozentsätze der Bestandteile stets 100 ist.

Bevorzugt ist, daß der Haftschmelzklebstoff zu 10 bis 50 Gew.% Styrol - Isopren - Styrol Synthesekautschuk, wie es bspw. im Handel unter der Bezeichnung CARIFLEX TR 1107® von der Fa. SHELL erhältlich ist; zwischen 10 und 80 Gew.% eines hydrierten Alkohols, wie er bspw. unter der Bezeichnung ABITOL® von der Fa. HERCULES erhältlich ist; zwischen 10 und 80 Gew.% eines Kohlenwasserstoffharzes, bspw. des Harzes HERCURES C® von der Fa. HERCULES, zwischen 1 und 40 Gew.% an Estern pflanzlicher Fettsäuren, bspw. MIGLYOL 812® von der Fa. DYNAMIT NOBEL und ggf. bis zu 5 Gew.% Alterungsschutzmittel, wie Hydrochinon etc. sowie bis zu 70 Gew.% Füllstoffe aufweist.

Bei einer weiteren bevorzugten Ausführungsform der Erfindung weist der Haftschmelzklebstoff zu 10 bis 50 Gew.% eines Polycaprolactons, bspw. CAPA 650® von der Fa. INTEROX; zwischen 10 und 80 Gew.% eines hydrierten Alkohols, bspw. ABITOL® von der Fa. HERCULES; zwischen 10 und 80 Gew.% eines Kohlenwasserstoffharzes, bspw. HERCURES C® von der Fa. HERCULES; zwischen 1 und 40 Gew.% an Estern pflanzlicher Fettsäuren, wie MIGLYOL 812® von der Fa.DYNAMIT NOBEL und ggf. bis zu 5 Gew.% Alterungsschutzmittel, sowie bis zu 70 Gew.% Füllstoffe auf.

Es kann auch bevorzugt sein, daß der Haftschmelzklebstoff zu 10 bis 50 Gew.% Polyethylen-Vinylacetat, wie EVATANE 28-25® von der Fa.ATOCHEM, zwischen 10 und 80 Gew.% eines hydrierten Alkohols, bspw. ABITOL® von der Fa. HERCULES, zwischen 10 und 80 Gew.% eines Kohlenwasserstoffharzes, bspw. des Harzes HERCURES C® von der Fa.HERCULES, zwischen 1 und 40 Gew.% an Estern pflanzlicher Fettsäuren bspw. MIGLYOL 812® von der Fa. DYNAMIT NOBEL und ggf. bis zu 5 Gew.% Alterungsschutzmittel, wie Hydrochinon etc., sowie bis zu 70 Gew.% Füllstoffe aufweist.

Ein geeigneter Haftschmelzklebstoff kann zu 10 bis 50 Gew.% Polyurethan; wie z.B. LUPHEN P 1110® der Fa. BASF zwischen 10 und 80 Gew.% eines hydrierten Alkohols, bspw. ABITOL® von der Fa. HERCULES zwischen 10 und 80 Gew.% eines Kohlenwasserstoffharzes, bspw. des Harzes HERCURES C® von der Fa. HERCULES zwischen 1 und 40 Gew.% an Estern pflanzlicher Fettsäuren, bspw. MIGLYOL 812® von der Fa. DYNAMIT NOBEL und ggf. bis zu 5 Gew.% Alterungsschutzmittel, sowie bis zu 70 Gew.% Füllstoffe aufweisen.

Es ist auch möglich, daß der Haftschmelzklebstoff zu 10 bis 50 Gew.% Polyamid, wie z.B. EURELON 930® der Fa. SCHERING; zwischen 10 und 80 Gew.% eines hydrierten Alkohols, bspw. ABITOL® von der Fa. HERCULES zwischen 10 und 80 Gew.% eines Kohlenwasserstoffharzes, bsw. des Harzes HERCURES C® von der Fa. HERCULES zwischen 1 und 40 Gew.% an Estern pflanzlicher Fettsäuren, bspw. MIGLYOL 812® von der Fa. DYNAMIT NOBEL und ggf. bis zu 5 Gew.% Alterungsschutzmittel, sowie bis zu 70 Gew.% Füllstoffe aufweist.

Es kann auch ein Haftschmelzklebstoff mit 10 bis 50 Gew.% Epoxid, bspw. z.B. EUREPOX 7001® der Fa. SCHERING, zwischen 10 und 80 Gew.% eines hydrierten Alkohols, bspw. ABITOL® von der Fa. HERCULES zwischen 10 und 80 Gew.% eines Kohlenwasserstoffharzes, bspw. des Harzes HERCURES C® von der Fa.HERCULES zwischen 1 und 40 Gew.% an Estern pflanzlicher Fettsäuren bspw. MIGLYOL 812® von der Fa.DYNAMIT NOBEL und ggf. bis zu 5 Gew.% Alterungsschutzmittel, wie Hydrochinon etc. sowie bis zu 70 Gew.% Füllstoffen verwendet werden.

Ein weiterer für die Herstellung erfindungsgemäßer Vorrichtungen verwendbarer Haftschmelzklebstoff weist zu 10 bis 50 Gew.% Polyisobuten mit zähklebriger kautschukartiger Konsistenz, wie z.B. OPPANOL B 50® der Fa. BASF, zwischen 10 und 80 Gew.% eines hydrierten Alkohols, bspw. ABITOL® von der Fa. HERCULES, zwischen 10 und 80 Gew.% eines Kohlenwasserstoffharzes, bspw. des Harzes HERCURES C® von der Fa.HERCULES, zwischen 1 und 40 Gew.% an Estern pflanzlicher Fettsäuren, bspw. MIGLYOL 812® von der Fa.DYNAMIT NOBEL und ggf. bis zu 5 Gew.% Alterungsschutzmittel, sowie bis zu 70 Gew.% Füllstoffe auf.

Schließlich ist es auch bevorzugt, Haftschmelzklebstoffe auf Polyesterbasis, die bspw. zwischen 10 und 80 Gew.% eines hydrierten Alkohols, bspw. ABITOL® von der Fa. HERCULES, zwischen 10 und 80 Gew.% eines Kohlenwasserstoffharzes, bspw. des Harzes HERCURES C® von der Fa.HERCULES zwischen 1 und 40 Gew.% an Estern pflanzlicher Fettsäuren bspw. MIGLYOL 812® von der Fa. DYNAMIT NOBEL und ggf. bis zu 5 Gew.% Alterungsschutzmittel, sowie bis zu 70 Gew.% Füllstoffe aufweisen, einzusetzen.

Erfindungsgemäß hergestellte Pflaster können ferner auch ein oder mehrere Nitroglycerin-Reservoirs, in dem/nen das Nitroglycerin mit gegenüber der wirkstoffaufweisenden Haftschmelzklebstoffschicht erhöhter Konzentration vorliegt, besitzen, wodurch höhere Nitroglycerindosen verarbeitet werden können und damit die Vorrichtung länger im Einsatz bleiben kann, bevor sie ausgewechselt werden muß. Typische Ausgestaltungen finden sich bspw. in der DE-A 36 29 304.0 (LOHMANN). Bevorzugte Ausgestaltungen der Erfindung sind in den Unteransprüchen aufgeführt, auf die hiermit ausdrücklich bezug genommen wird.

Die Herstellung der erfindungsgemäß notwendigen Schmelzen erfolgt nach an sich bekannten Verfahren. Bei der Verarbeitung von Nitroglycerin als explosivem und flüchtigem Stoff sind nachfolgende besondere Maßnahmen zur Verarbeitung angezeigt:
A) Arbeiten bei möglichst tiefen Temperaturen;
B) Erhöhung des Außendrucks durch bekannte Maßnahmen;
C) Sättigung des Dampfraums über der Schmelze mit dem dampfförmigen Nitroglycerin, wie Arbeiten in gekapseltem System, und
D) Arbeiten bei kleinstmöglichem Nitroglycerinanteil in der Schmelze.

Es ist hier sinnvoll, in einem geschlossen System zu arbeiten, um ein ungeregeltes Verdampfen des Nitroglycerins und ggf. ein Ansammeln explosiver Mengen desselben an kühlen Bereichen der Anlage, wie bspw. in Abzugsanlagen, zu vermeiden.

Weitere Merkmale und Vorteile der Erfindung werden nachfolgend anhand der begleitenden Zeichungen erläutert.

Dabei zeigt:
- Fig.1: schematisch einen Schnitt durch eine nach der Erfin dung hergestellte Vorrichtung mit Nitroglycerindepot;
- Fig.2: einen schematisch dargestellten Schnitt durch eine weitere erfindungsgemäße Vorrichtung mit Nitroglycerindepot; und
- Fig.3: einen schematisch dargestellten Schnitt durch eine weitere Ausführungsform einer Vorrichtung ohne Nitroglycerindepot.

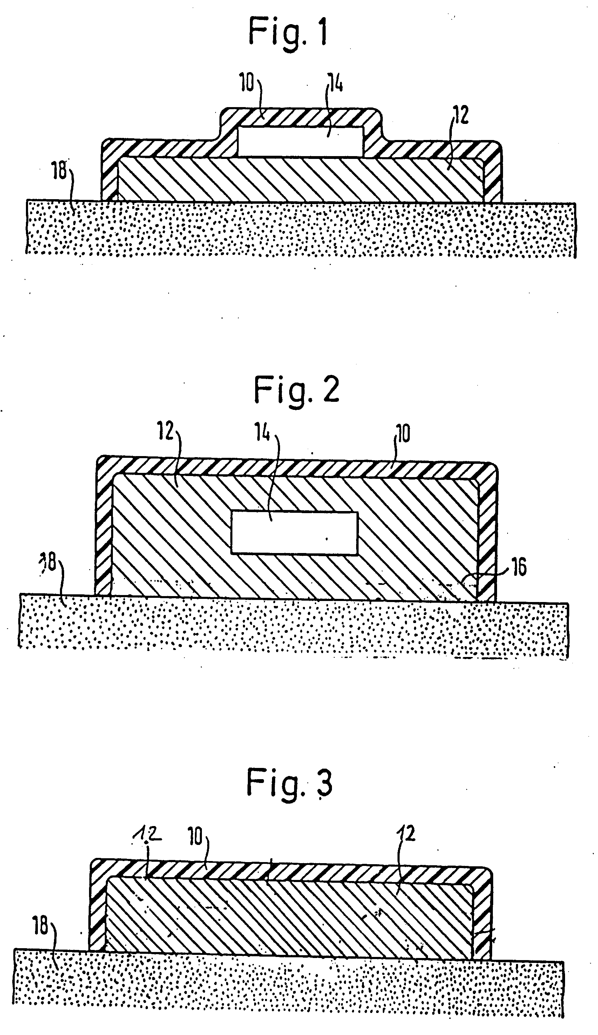

In Fig. 1 ist eine bevorzugte Ausführungsform eines erfindungsgemäß hergestellten Pflasters als transdermales System zur Abgabe von Nitroglycerin an die Haut gezeigt. Sie besitzt einen nitroglycerindurchlässigen Haftschmelzkleber 12, ein Nitroglycerindepot 14, in dem das Nitroglycerin zumindest eine höhere Konzentration als im Haftschmelzkleber 12 aufweist sowie eine Nitroglycerin undurchlässige Rückschicht 10, auf der das Nitroglycerindepot 14 aufliegt.

Das Pflaster ist auf die Haut 18 aufgeklebt.

Es wandert nun kontinuierlich Nitroglycerin mit vorherbestimmter Rate durch den Haftschmelzkleber in die Haut 18, wodurch der Nitroglyceringehalt im Haftschmelzkleber 12 abnimmt. Die Nitroglycerinabnahme wird durch Nachströmen von Nitroglycerin aus dem Nitroglycerindepot 14 kompensiert, so daß über einen vorherbestimmten Zeitraum eine vorherbestimmbare Gleichgewichtskonzentration des Nitroglycerins im Haftschmelzkleber 12 herrscht, die für die Abgabe konstanter Mengen Nitroglycerin an die Haut 18 sorgt.

In Fig.2 ist eine weitere bevorzugte Ausführungsform eines erfindungsgemäß hergestellten Pflasters dargestellt, bei der ein Nitroglycerindepot 14 allseitig vom Haftschmelzkleber 12 umgeben ist. Diese Ausführungsform eignet sich insbesondere dann, wenn eine große Kontaktfläche Nitroglycerindepot/Haftschmelzkleber für schnelle Nitroglycerinabgabe an den Haftschmelzkleber erwünscht ist.

Fig. 3 ist eine weitere einfache Ausführungsform eines erfindungsgemäß hergestellten Pflasters dargestellt, bei dem auf einem nitroglycerinundurchlässigen Rückschichtmaterial 10 ein nitroglycerinhaltiger Haftschmelzkleber 12 derart aufgebracht ist, daß das Rückschichtmaterial 10 den Haftschmelzkleber 12 dreiseitig abdeckt. Mit der freien Haftschmelzklebefläche ist diese auf die Haut 18 aufgeklebt, so daß ein ganzflächiger Kontakt über die gesamte Applikationsfläche gewährleistet ist und der Übergang des Nitroglycerins vom Haftschmelzkleber an die Haut stets über eine gleichbleibende Fläche mit gleichbleibender Geschwindigkeit erfolgt.

Nachfolgend soll die erfindungsgemäß verbesserte Herstellung eines nitroglycerinhaltigen transdermalen Systems beschrieben werden.

Zunächst wird eine Mischung der Komponenten des Haftschmelzklebers mit Nitroglycerin hergestellt. Diese Mischung wird auf Verarbeitungstemperatur gebracht und aus der Schmelze auf ein Rückschichtmaterial aufgetragen. Die weitere Verarbeitung, wie das Aufbringen eines abhäsiv ausgerüsteten Schutzschichtmaterials erfolgt in üblicher Weise. Dabei kann das Nitroglycerin sowohl in einer Lösung, bevorzugt gelöst in einem der Bestandteile des Haftschmelzklebers oder aber auch an einem Trägermaterial, wie Lactose, absorbiert in die Haftschmelzklebermasse eingearbeitet werden.

Ein besonderer Vorteil des erfindungsgemäßen Verfahrens besteht darin, daß sogleich nach Aufbringen des Haftschmelzklebers, bspw. über eine Breitschlitzdüse, das Träger- bzw. Schutzschichtmaterial zukaschiert werden kann, so daß durch Abdecken der Haftschmelzkleberschicht die Verdampfung des Nitroglycerins im frisch hergestellten System unterdrückt wird.

Nachfolgend wird nun die Herstellung eines Nitroglycerinpflasters beschrieben:

### Beispiel:

15,5 g (22,24 Gew.%) eines Ethylen-Vinylacetat-Copolymeren, wie es im Handel unter der Bezeichnung Evatane 28-25® von der Firma Atochem erhältlich ist, werden mit 20,4 g (29,26 Gew.%) Hydroabietylalkohol, wie er unter der Bezeichnung ABITOL® von der Fa. Hercules im Handel erhältlich ist, 30,4 g (43,6 Gew.%) eines Tackifiers auf Basis aliphatischer Kohlenwasserstoffharze, wie er unter der Bezeichnung Hercures® (Fa. Hercules) im Handel erhältlich ist, sowie 3,4 g (4,88 Gew.%) eines Weichmachers auf der Basis von Estern mittelkettiger pflanzlicher Fettsäuren (im Handel unter der Bezeichnung MIGLYOL 812® von der Fa. Dynamit Nobel erhältlich) vermischt und auf eine Temperatur von 110°C zum Schmelzen erhitzt. Nach Abkühlen auf 50 bis 60°C werden zu dieser Mischung unter Rühren 26,6 g Nitroglycerin-Lactose-Verreibung (entsprechend 2,7 g reinen Nitroglycerins) gegeben. Diese Mischung wird auf eine einseitige mit Aluminium bedampfte und silikonisierte Polyesterfolie geschichtet (Flächengewicht des Kleberfilmes: 402 g/m² ) und eine aluminisierte Polyesterfolie aufkaschiert. Das derart erhaltene Schichtmaterial wurde in 16 cm² große rechteckige Pflaster geschnitten und in an sich bekannter Weise in Siegelbeutel einzeln verpackt.

Selbstverständlich sind auch beliebige andere Nitroglycerin aufweisende transdermale therapeutische Systeme erfindungsgemäß unter Einsatz von Haftschmelzklebern herstellbar, so daß die oben aufgeführten Beispiele keineswegs den Schutzbereich einschränken sollen, sondern lediglich den vielfältigen vorteilhaften Einsatz von Haftschmelzklebern in diesem Gebiet verdeutlichen sollen.

## Patentansprüche

1. Verfahren zur Herstellung eines Pflasters zur gesteuerten Abgabe von Nitroglycerin an die menschliche oder tierische Haut mit einer für das Nitroglycerin undurchlässigen Rückschicht und einem haftklebenden Nitroglycerin enthaltenden Reservoir, gekennzeichnet durch Herstellung einer Mischung aus geschmolzenem Haftschmelzkleber mit einer Verarbeitungstemperatur von 40 bis 80°C und Nitroglycerin und Aufbringen der Mischung auf eine Unterlage.

2. Verfahren nach Anspruch 1, dadurch gekennzeichnet, daß die Verarbeitungstemperatur des Haftschmelzklebers zwischen 40 und 60°C und bevorzugt zwischen 40 und 55°C liegt.

3. Verfahren nach einem der vorangehenden Ansprüche, dadurch gekennzeichnet, daß der Haftschmelzkleber auf der Basis von Styrol-Isopren-Styrol-Blockpolymeren, Polycaprolactonen, Ethylen-Vinylacetat-Copolymeren, Polyurethan, Polyepoxiden, Polyisobuten, Polyvinylethern, gegebenenfalls unter Zusatz von Weichmachern, Tackifiern, Füllstoffen, Alterungsschutzmitteln und/oder Thixotropiermitteln hergestellt ist.

4. Verfahren nach einem der vorangehenden Ansprüche, dadurch gekennzeichnet, daß der Haftschmelzkleber aus zwischen 10 und 80 Gew.%, bevorzugt 20 bis 80 Gew.% und besonders bevorzugt 20 bis 50 Gew.% Polymer, zwischen 1 und 80 Gew.% Weichmachern, zwischen 10 und 80 Gew.%, bevorzugt 15 bis 60 Gew.% Tackifier, gegebenenfalls 0,1 bis 5 Gew.% Alterungsschutzmitteln und gegebenenfalls 0 bis 70 Gew.% Füllstoffen hergestellt wird, wobei die Summe der Prozentsätze der Bestandteile stets 100 ist.

5. Verfahren nach einem der vorangehenden Ansprüche, gekennzeichnet durch kontinuierliches oder diskontinuierliches Aufbringen nitroglycerinhaltigen geschmolzenen Haftschmelzklebers bei einer Temperatur des Haftschmelzklebers zwischen 40 und 80, bevorzugt 40 bis 60°C und besonders bevorzugt 45 bis 55°C, wobei die Unterlage ein Trägermaterial ist.

6. Verfahren zur Herstellung eines Pflasters nach einem der Ansprüche 1 bis 4, gekennzeichnet durch kontinuierliches oder diskontinuierliches Aufbringen nitroglycerinhaltigen geschmolzenen Haftschmelzklebers bei einer Temperatur des Haftschmelzklebers zwischen 40 und 80 °C, bevorzugt 40 bis 60 °C und besonders bevorzugt 40 bis 55 °C, wobei die Unterlage ein Schutzschichtmaterial ist.

7. Verfahren zur Herstellung eines Pflasters nach einem der vorangehenden Ansprüche, dadurch gekennzeichnet, daß das Auftragen des Haftschmelzklebers mit einer Verarbeitungstemperatur zwischen 40 und 80°C durch Extrusion, Gießen, Walzenauftrag, Rakelauftrag, Aufsprühen oder ein Druckverfahren erfolgt.

8. Verfahren nach Anspruch 7, dadurch gekennzeichnet, daß der das Nitroglycerin aufweisende Haftschmelzkleber als eine oder mehrere Schichten aufgebracht wird.

9. Verfahren nach einem der vorangehenden Ansprüche 1 bis 5 und 7 bis 8, dadurch gekennzeichnet, daß eine ablösbare Schutzschicht aufgebracht wird.

## Claims

1. Process of producing a plaster for the controlled delivery of nitroglycerine to human or animal skin, said plaster having a backing layer, which is impermeable to nitroglycerine, and an adhesive reservoir containing nitroglycerine, characterised by producing a mixture formed from molten hot melt pressure sensitive adhesive, having a processing temperature of between 40 and 80°C, and nitroglycerine, and by applying the mixture to a base.

2. Process according to claim 1, characterised in that the processing temperature of the pressure sensitive adhesive lies between 40 and 60°C and preferably between 40 and 55°C.

3. Process according to one of the preceding claims, characterised in that the pressure sensitive adhesive is produced on the basis of styrene-isoprene-styrene block polymers, polycaprolactones, ethylene-vinylacetate copolymers, polyurethane, polyepoxides, polyisobutene, polyvinyl ethers, possibly with the addition of plasticizers, tackifiers, filler materials, anti-agens and/or thixotropic agents.

4. Process according to one of the preceding claims characterised in that the pressure sensitive adhesive is produced from between 10 and 80% by wt., preferably 20 25 to 80% by wt. and especially preferably 20 to 50% by wt., polymer, between 1 and 80% by wt. plasticizer, between 10 and 80% by wt., preferably 15 to 60 % by wt., tackifier possibly 0.1 to 5% by wt. anti-oxidant, and possibly 0 to 70% by wt. filler materials, the sum of the percentages of the constituent ingredients always being 100.

5. Process according to one of the preceding claims, characterised by continuously or discontinously applying nitroglycerine-containing molten hot melt pressure sensitive adhesive at a temperature of the pressure sensitive adhesive of between 40 and 80°C, preferably 40 to 60°C and especially preferably 45 to 55°C, the base being a carrier material.

6. Process of producing a plaster according to one of claims 1 to 4, characterised by continuously or discontinuously applying nitroglycerine-containing molten hot melt pressure sensitive adhesive at a temperature of the pressure sensitive adhesive of between 40 and 80°C, preferably between 40 to 60°C and especially preferably 40 to 55°C, the base being a protective layer material.

7. Process of producing a plaster according to one of the preceding claims, characterised in that the hot melt pressure sensitive adhesive, having a processing temperature of between 40 and 80°C, is applied by extrusion, moulding, roller application, wiper application, spraying or by a printing process.

8. Process according to claim 7, characterised in that the nitroglycerine-containing hot melt pressure sensitive adhesive is applied as one or a plurality of layers.

9. Process according to one of the preceding claims 1 to 5 and 7 to 8, characterised in that a detachable protective layer is applied.

## Revendications

1. Procédé de préparation d'un pansement à libération retardée de nitroglycérine sur la peau humaine ou animale, comportant une couche postérieure imperméable à la nitroglycérine et un réservoir autocollant contenant de la nitroglycérine, le procédé étant caractérisé par la préparation d'un melange de colle adhésive fusible fondue avec une température de traitement de 40 à 80°c et de nitroglycérine et par l'application du mélange sur une base.

2. Procédé selon la revendication 1, caractérisé en ce que la température de traitement de la colle fusible est comprise entre 40 et 60°C, de préference entre 40 et 55°C.

3. Procedé selon l'une quelconque des revendications précédentes, caractérisé en ce que la colle adhésive fusible est préparée à base de polymères-blocs de styrène-isoprène-styrène, de polycaprolactones, de copolymères d'éthylène et d'acétate de vinyle, de polyuréthane, de polyépoxydes, de polyisobutène, d'éthers de polyvinyle, éventuellement avec addition de plastifiants, d'adhésivants, de charges, d'agents anti-vieillissement et/ou d'agents thixotropes.

4. Procédé selon l'une quelconque des revendications précédentes, caractérisé en ce que la colle adhésive fusible est préparée en utilisant entre 10 et 80 % en poids, de préférence entre 20 et 80 % en poids, mieux encore entre 20 et 50 % en poids de polymère, entre 1 et 80 % en poids de plastifiants, entre 10 et 80% en poids, de préférence entre 15 et 60 % en poids d'adhésivant éventuellement 0,1 à 5 % en poids d'agents antivieillissement et éventuellement 0 à 70 % en poids de charges, la somme des pourcentages des composants étant toujours de 100.

5. Procédé selon l'une quelconque des revendications précédentes, caractérié par l'application continue ou discontinue de colle adhésive fusible fondue contenant de la nitroglycerine à une temperature de la colle comprise entre 40 et 80°C, de préférence entre 40 et 60°C,mieux encore entre 45 et 55°C, la base étant une matière de support.

6. Procédé de préparation d'un pansement selon l'une quelconque des revendications 1 à 4, caractérisé par l'application continue ou discontinue de colle fusible fondue contenant de la nitroqlycérine à une température de la colle comprise entre 40 et 80°C, de préférence entre 40 et 60°C, mieux encore entre 40 et 55°C, la base étant une matière en couche de protection.

7. Procédé de préparation d' un pansement selon l'une quelconque des revendications précédentes, caractérisé en ce que l'application de la colle adhésive fusible dont la température de traitement est comprise entre 40 et 80°C se fait par extrusion, coulée, application au rouleau, application a la lame, pulvérisation ou impression .

8. Procédé selon la revendication 7, caractérisé en ce que la colle adhésive fusible contenant la nitroglycérine est appliquée en une ou plusieurs couches.

9. Procédé selon l'une quelconque des revendications 1 a 5 et 7 à 8, caractérisé en ce que l'on applique une couche de protection détachable.
